(19) Europäisches Patentamt
European Patent Office
Office européen des brevets

(11) **EP 2 092 928 A1**

(12) **EUROPÄISCHE PATENTANMELDUNG**

(43) Veröffentlichungstag:
26.08.2009 Patentblatt 2009/35

(21) Anmeldenummer: 08003246.9

(22) Anmeldetag: 22.02.2008

(51) Int Cl.:
*A61K 8/04* *(2006.01)*     *A61K 8/29* *(2006.01)*
*A61K 8/35* *(2006.01)*     *A61K 8/41* *(2006.01)*
*A61K 8/46* *(2006.01)*     *A61K 8/49* *(2006.01)*
*A61Q 17/04* *(2006.01)*

(84) Benannte Vertragsstaaten:
AT BE BG CH CY CZ DE DK EE ES FI FR GB GR
HR HU IE IS IT LI LT LU LV MC MT NL NO PL PT
RO SE SI SK TR
Benannte Erstreckungsstaaten:
AL BA MK RS

(71) Anmelder: **STADA ARZNEIMITTEL AG**
**61118 Bad Vilbel (DE)**

(72) Erfinder:
• **HANSEN, Peter Dr.**
**63303 Dreieich (DE)**

• **HEPPNER, Andrea**
**61197 Florstadt (DE)**
• **RILLMANN, Thomas Dr.**
**76756 Bellheim (DE)**
• **SCHUMANN, Christof**
**35767 Breitscheid- Erdbach (DE)**

(74) Vertreter: **Wittkopp, Alexander**
**Maiwald Patentanwalts GmbH**
**Jungfernstieg 38**
**20354 Hamburg (DE)**

(54) **Lichtschutzzubereitung in Form einer Hydrodispersion mit einem Lichtschutzfaktor von _> 50**

(57)     Die Erfindung betrifft Lichtschutzzubereitungen in Form einer Hydrodispersion, die einen Lichtschutzfaktor von ≥ 50 aufweisen und mindestens einen öllöslichen UV-Filter, mindestens einen wasserlöslichen UV-Filter sowie mindestens einen pigmentären UV-Filter enthalten, vorzugsweise emulgatorfrei sind sowie geeignet sind zur Anwendung bei Sonnenallergie und/oder Mallorca-Akne.

EP 2 092 928 A1

**Beschreibung**

Die Erfindung betrifft Lichtschutzzubereitungen in Form einer Hydrodispersion, die einen Lichtschutzfaktor von ≥ 50 aufweisen und mindestens einen öllöslichen UV-Filter, mindestens einen wasserlöslichen UV-Filter sowie mindestens einen pigmentären UV-Filter enthalten, vorzugsweise emulgatorfrei sind sowie geeignet sind zur Anwendung bei Sonnenallergie und/oder Mallorca-Akne.

Die schädigende Wirkung des ultravioletten Teils der Sonnenstrahlung auf die Haut ist seit langem bekannt. Derartige Schädigungen können eine einfache Hautreizung, z.B. ein leichter Sonnenbrand sein, können sich aber auch in Zellschädigungen manifestieren. Es ist bekannt, dass vor allem durch UV-B-Strahlung (das ist Strahlung mit einer Wellenlänge zwischen 290 nm und 320 nm) in der Haut DNS-Schäden hervorgerufen werden können, die zu Zellmutationen und somit zu Hautkrebs führen.

Auch werden die vorzeitige Hautalterung, die Bildung von Falten und die Erschlaffung des Hautbindegewebes durch UV-Strahlung beschleunigt; verantwortlich hierfür ist vor allem die langwellige UV-A-Strahlung (das ist Strahlung mit einer Wellenlänge zwischen 320 nm und 400 nm). Auch sie begünstigt die Auslösung einer Erythembildung oder verstärkt diese Reaktion bei manchen Personen, und sie kann sogar die Ursache für durch Licht ausgelöste toxische oder allergische Reaktionen sein.

Zur Vorbeugung gegen derartige Schädigungen sind in den letzten Jahren immer effektivere Sonnenschutzmittel entwickelt worden, in denen zahlreiche physikalische und chemische Filtersubstanzen die Haut vor UV-A-Strahlung und vor UV-B-Strahlung schützen sollen.

Physikalische Lichtschutzfilter, wie z.B. Oxide von Metallen, wirken auf der Haut verteilt wie kleine Spiegel, die die UV-Strahlung reflektieren und streuen.

Durch chemische Lichtschutzfilter wird die auf die Haut auftreffende UV-Strahlung in Wärmeenergie umgewandelt. Chemische Lichtschutzfilter können sowohl lipophile als auch hydrophile Eigenschaften aufweisen und entsprechend dieser Eigenschaften unterscheidet man zwischen öllöslichen und wasserlöslichen Filtern.

Lichtschutzzubereitungen werden oftmals als Emulsionen, Lotionen oder Gele formuliert.

Aufgabe der vorliegenden Erfindung war es, gegenüber dem Stand der Technik verbesserte Lichtzusammensetzungen anzugeben.

Insbesondere war es Aufgabe der vorliegenden Erfindung, Lichtschutzzubereitungen in Form einer Hydrodispersion anzugeben, die einen Lichtschutzfaktor von ≥ 50 aufweisen.

Durch umfangreiche Untersuchungen hat die Anmelderin in unerwarteter und überraschender Weise festgestellt, dass die Kombination von öllöslichen und wasserlöslichen UV-Filtern mit pigmentären UV-Filtern die Erzielung von Liehtschutzzubereitungen erlaubt, die als Hydrodispersionen vorliegen und einen Lichtschutzfaktor von ≥ 50 aufweisen. Sie hat des Weiteren überraschend gefunden, dass stabile Hydrodispersionen mit derartig hohen Lichtschutzfaktorwerten durch Einarbeitung mindestens eines öllöslichen UV-Filters, mindestens eines wasserlöslichen UV-Filters sowie mindestens eines pigmentären UV-Filters auch erzielt werden können, wenn der Emulgatorgehalt und/oder Fettgehalt der Zusammensetzungen sehr niedrig ist oder die Zusammensetzungen sogar fett- und/oder emulgatorfrei sind.

Gegenstand der vorliegenden Erfindung ist zum einen eine Hydrodispersion mit einem Lichtschutzfaktor ≥ 50, enthaltend mindestens einen öllöslichen UV-Filter, mindestens einen wasserlöslichen UV-Filter sowie mindestens einen pigmentären UV-Filter.

In bevorzugten Ausführungsformen ist der Emulgatorgehalt in den erfindungsgemäßen Lichtschutzzubereitungen ≤ 2 Gew.-%, bevorzugt ≤ 0,2 Gew.-% und besonders bevorzugt ≤ 0,01 Gew.-%.

In weiteren bevorzugten Ausführungsformen ist der Fettgehalt ≤ 1 Gew.-%, bevorzugt ≤ 0,1 Gew.-% und besonders bevorzugt ≤ 0,01 Gew.-%.

In einer besonders bevorzugten Ausführungsform ist die erfindungsgemäße Lichtschutzzubereitung fett- und/oder emulgatorfrei.

Weiterhin bevorzugt enthält die erfindungsgemäße Lichtschutzzubereitung zusätzlich mindestens einen UV-A-Filter, bevorzugt Diethylamino Hydroxybenzoyl Hexyl Benzoate (Uvinul A Plus der Firma BASF) und/oder Butylmethoxydibenzoylmethane (Parsol 1789 von DSM bzw. Neo Heliopan 357 von Symrise) und/oder Bis-Ethylhexyloxyphenol Methoxyphenyl Triazine (Tinosorb S von der Firma Ciba) und/oder anorganische Pigmente wie Zinkoxid.

Es wurde überraschend gefunden, dass die Verwendung der UV-A-Filter Butylmethoxydibenzoylmethane und/oder Diethylamino Hydroxybenzoyl Hexyl Benzoate und/oder Bis-Ethylhexyloxyphenol Methoxyphenyl Triazine zu einer Steigerung der UV-B-Schutzwirkung der erfindungsgemäßen Lichtschutzzubereitungen führt.

Die UV-A-Filter sind in den erfindungsgemäßen Zusammensetzungen jeweils bevorzugt in Mengen von 0,2 bis 10 Gew.-%, insbesondere von 0,5 bis 8 Gew.-% enthalten,

Es wurde des Weiteren überraschend gefunden, dass erfindungsgemäße Hydrodispersionsgele mit einem Lichtschutzfaktor ≥ 50, die vorzugsweise einen niedrigen Fett- und/oder Emulgatorgehalt aufweisen und besonders bevorzugt fett- und/oder emulgatorfrei sind, einen UV-A-Schutzfaktor ≥ 20 und eine UV-A-/UV-B-Ratio ≥ 1/3 aufweisen, wenn sie des Weiteren wenigstens einen UV-A-Filter, vorzugsweise den/die UV-Filter Butylmethoxydibenzoylmethane und/oder Diethylamino Hydroxybenzoyl Hexyl Benzoate und/oder Bis-Ethylhexyloxyphenol Methoxyphenyl Triazine enthalten.

Der UV-A-Schutzfaktor wird entweder nach der in-vivo-PPD-Methode bestimmt ("Persistent pigment darkening method in der von der französischen Gesundheitsagentur Agence francaise de sécurité sanitaire des produits de santé - Afssaps geänderten Form"), oder nach der in-vitro-COLIPA-Methode bestimmt ("In vitro UVA test method gemäß COLIPA Recommendation No. 20").

Die UV-A/UV-B-Ratio ist das Verhältnis des UV-A-Schutzfaktors zum UV-B-Schutzfaktor.

Die erfindungsgemäßen Lichtschutzzubereitungen eignen sich gemäß weiterhin bevorzugten Ausführungsformen zum Schutz von gegenüber Sonnenallergie und/oder Mallorca-Akne empfindlicher bzw. von Sonnenallergie und/oder Mallorca-Akne betroffener Haut vor Sonnenstrahlung, insbesondere UV-Strahlung, das heißt die erfindungsgemäßen Lichtschutzzubereitungen sind für die Verwendung bei Sonnenallergie und/oder Mallorca-Akne geeignet.

Ein weiterer Gegenstand der vorliegenden Erfindung ist somit die Verwendung der erfindungsgemäßen Lichtschutzzubereitungen zum Schutz von gegenüber Sonnenallergie und/oder Mallorca-Akne empfindlicher bzw. von Sonnenallergie und/oder Mallorca-Akne betroffener Haut vor Sonnenstrahlung, insbesondere UV-Strahlung,

Hydrodispersionen stellen eine übliche Formulierungsgrundlage für Sonnenschutzzubereitungen dar. Sie lassen sich gut auf die Haut auftragen und ziehen schnell ein. Bisher war es aber nicht möglich, stabile Hydrodispersionen mit einem Lichtschutzfaktor ≥ 50 herzustellen. Insbesondere war es nicht möglich, hohe Lichtschutzwerte von ≥ 50 in Hydrodispersionen zu erzielen, die nur geringe Gehalte an Emulgatoren und/oder Fetten aufweisen, insbesondere fett- und emulgatorfrei sind und für die Verwendung bei Sonnenallergie und/oder Mallorca-Akne geeignet sind.

Der Lichtschutzfaktor ist die Maßzahl, mit der die Wirksamkeit einer Lichtschutzformulierung gemessen wird und gibt an, wie viel länger man sich mit der auf die Haut aufgetragenen Lichtschutzformulierung der Sonne aussetzen kann, als dies mit der jeweils individuellen Eigenschutzzeit möglich wäre, bis ein Sonnenbrand entsteht. Wird vor und nach Auftragen eines Lichtschutzpraparates die minimale Erythemdosis (MED), das heißt die Menge an UV-B-Bestrahlung, die ein gerade erkennbares Erythem induziert, bestimmt, ergibt sich der Lichtschutzfaktor (LSF) nach folgender Formel:

$$\text{LSF} = (\text{MED mit Sonnenschutzmittel}) \, / \, (\text{MED ohne Sonnenschutzmittel})$$

Der Lichtschutzfaktor wird nach der "International Sun Protection Factor Method (2006)" bestimmt.

Als erfindungsgemäße öllösliche und wasserlösliche UV-Filter sind insbesondere die folgenden zu nennen;

p-Aminobenzoesäurederivate, z.B. 4-Aminobenzoesäure, 4-Dimethylaminobenzoesäure-2.ethylhexylester (Eusolex 6007) oder ethoxyliertes Ethyl-4-aminobenzoat (Uvinul P25),

Salicylsäurederivate, z.B. 3,3,5-Trimethyl-cyclohexyl-salicylat (Neo Heliopan HMS) oder Salicylsäure-2-ethylhexylester (Neo Heliopan OS),

Benzophenonderivate, z.B. 2-Hydroxy-4-methoxy-beazophenon (Neo Heliopan BB), 2-Hydroxy-4-methoxy-benzophenon-5-sulfonsäure und ihr Natriumsalz (Uvinul MS 40) oder 2-[4-(Dielhylamino)-2-hydroxybenzoyl]-Benzoosäurehexylester (Uvinul A Plus),

Sulfonsäurederivate, z.B. 2-Phenylbenzimidazol-5-sulfonsäure und ihre Kalium-, Natrium- und Triethanolaminsalze (Neo Heliopan Hydro) oder Benzol-1,4-di(3-methyliden-10-camphersulfonsäure) (Mexoryl SX),

Dibenzoylznethanderivate, z.B. 1-(4-tert-Butylphenyl)-3-(4-methoxyphenyl)-propan-1,3-dion (Parsol 1789),

Diphenylacrylate, z.B. 2-Cyano-3,3-diphenylacrylsäure-2-ethylhexylester (Neo Heliopan 303),

Campherderivate, z.B. 3-(4'-Trimethylammonium)-benzyliden-boman-2-on-methylsulfat (Mexoryl SO), 3-(4'-Sulfo)-benzyliden-boman-2-on und seine Salze (Mexoryl SL) oder 3-(4'-Methylbenzyliden)-DL-campher (Eusolex 6300) oder 3-Benzylidencampher (Mexoryl SDS20),

Triazinderivate, z.B. 2,4,6-Tris[p-(2-ethylhexyl-oxycarbonyl)anilino] 1,3,5-triazin (Uvinul T 150), 4,4'-[(6-[4-((1,1 -Dimethylethyl)arninocarbonyl)phenyl-amino)-1,3,5-triazin-2,4-diyl)diimino]bis(benzoesäure-2-ethylhexylester) (Uvasorb HEB) oder 2,4-Bis[4-(2-ethylhexyloxy)-2-hydroxyphenyl]-6-(4-methoxyphenyl)-1,3,5-triazin (Tinosorb S),

Benzotriazolderivate, z.B. 2-(2H-Benzotriazol-2-yl)-4-methyl-6-(2-methyl-3-(1,3,3,3-tetramethyl-1-(trimethylsilyloxy)disiloxanyl)propyl)phenol (Mexoryl XL),

Benzimidazolderivate, z.B. 2,2'-(1,4-Phenylen)bis(1H-benzimidazol-4,6-disulfonsäure, Mononatriumsalz) (Neo Heliopan AP),

Benzoxazol-Derivate, z.B. 2,4-Bis-[5-1(dimethylpropyl)benzoxazol-2-yl-(4-phenyl)-imino]-6-(2-ethylhexyl)-imino-1,3,S-triazin (Uvasorb K2A),

Polymer von N-[2(und 4)-(2-oxobom-3-ylidenmethyl)benzyl]acrylamid (Mexoryl SW,

3-(4-(2,2-bis Ethoxyoarbonylvinyl)-phenoxy)propenyl)-methoxysiloxan/Dimethylsiloxan-copolymer (Parsol SLX).

Erfindungsgemäß sind diese organischen UV-Filter jeweils bevorzugt in Mengen von 0,1 bis 10 Gew.-%, vorzugsweise von 0,5 bis 8 Gew.-%, insbesondere 1 bis 7 Gew.-% in den erfindungsgemäßen Zusammensetzungen enthalten.

Vorzugsweise werden mehrere verschiedene öllösliche und wasserlösliche UV-Filter kombiniert. Erfindungsgemäß liegen aber mindestens ein öllöslicher und mindestens ein wasserlöslicher UV-Filter in den erfindungsgemäßen Lichtschutzzubereitungen vor.

Des Weiteren enthalten die erfindungsgemäßen Hydrodispersionen mindestens einen pigmentären UV-Filter, wobei es sich um anorganische pigmentäre UV-Filter handeln kann.

Vorteilhafte anorganische pigmentäre UV-Filter sind Metalloxide, insbesondere Oxide des Titans oder Zinks. Wenn anorganische Pigmente enthalten sind, werden diese vorzugsweise in mikronisierter Form eingesetzt, vorzugsweise in mittleren Teilchengrößen < 100 μm, besonders bevorzugt in mittleren Teilchengrößen zwischen 10 nm und 200 nm, Sie sind dann bevorzugt in Mengen von 0,1 bis 25 Gew.-%, insbesondere 0,5 bis 12 Gew.-% enthalten. Dabei ist es besonders vorteilhaft, wenn die anorganischen Pigmente in hydrophober Form vorliegen, d.h., dass sie oberflächig wasserabweisend ausgestaltet sind. Dies kann z.B. dadurch erfolgen, dass die Pigmentteilchen mit einer hydrophoben Oberflächenschicht aus Silikon überzogen werden. Derartige Pigmente sind kommerziell erhältlich beispielsweise von der Firma Tayca unter der Hasidelsbezeichnung MT 100 T, MT 100 Z und von der Degussa unter der Bezeichnung T 805 sowie von der Firma Franken Chemie unter der Bezeichnung Tego Sun T 805.

Die pigmentären UV-Filter werden vorzugsweise in einer Menge von 0,1 bis 25 Gew.-%, besonders bevorzugt von 0,5 bis 12 Oew.-% eingesetzt.

Vorzugsweise liegt das Verhältnis von löslichen zu pigmentären UV-Filtern in den erfindungsgemäßen Zusammensetzungen im Bereich von 0,2 bis 10, insbesondere von 0,5 bis 5.

Das Verhältnis der wasserlöslichen zu den öllöslichen UV-Filtern liegt in den erfindungsgemäßen Zusammensetzungen vorzugsweise im Bereich von 0,02 bis 2, insbesondere von 0,1 bis 1.

Vorzugsweise sind die erfindungsgemäßen Lichtschutzzubereitungen, insbesondere, wenn sie mindestens einen UV-A-Filter enthalten, frei von UV-Filtern des Cinnamat-Typs, insbesondere frei von 4-Methoxyzimtsäure-2-ethylhexylester (Neo Heliopan AV) und/oder 4-Methoxyzimtsäure Isoamylester (Neo Heliopan E1000).

Die erfindungsgemäßen Hydrodispersionen umfassen grundsätzlich eine Ölphase, ein geeignetes Verdickungsmittel, Wasser sowie einen wirksamen Gehalt an der erfindungsgemäßen Wirkstoffkombination. Die Ölphase wird aus den öligen bzw. öllöslichen UV-Filtern und ggf. weiteren öligen Bestandteilen gebildet. Sie enthält gemäß bevorzugten Ausführungsformen aber nur einen sehr geringen und vorzugsweise keinen Anteil an Fetten, fetten Ölen oder vergleichbaren Bestandteilen, die zu einer Auslösung der Sonnenallergie und/oder Mallorca-Akne beitragen könnten.

Fette sind Ester des dreiwertigen Alkohols Glycerin (Propan-1,2,3-triol) mit drei, meist verschiedenen, überwiegend geradzahligen und unverzweigten aliphatischen Monocarbonsäuren, den sogenannten Fettsäuren. Bei Raumtemperatur flüssige Fette, das heißt Gemische von Fettsäuretriglyceriden, werden oft als fette Öle bezeichnet. Die Ölphase der erfindungsgemäßen Hydrodispersionen enthält in besonders bevorzugten Ausführungsformen keine derartigen Fette bzw. fetten Öle.

Besonders bevorzugt sind die erfindungsgemäßen Zusammensetzungen frei von die Hautverträglichkeit potentiell negativ beeinflussenden Zusatzstoffen, insbesondere enthalten sie keine PEG-Derivate, Konservierungsstoffe oder Duftstoffe. Vorzugsweise sind die Zubereitungen wasserfest.

Die folgenden Ausführungsbeispiele sollen die vorliegende Erfindung veranschaulichen, ohne sie zu beschränken. Die Inhaltsstoffe sind gemäß INCI-Nomenklatur bezeichnet worden.

Beispiel 1: Emulgatorfreies Hydrodispersionsgel mit einem Lichtschutzfaktor ≥ 50

| INCI | g/100 g |
|---|---|
| Aqua | 34,05 |
| Disodium EDTA | 0,10 |
| Xanthan Gum | 0,50 |
| Pentylene Glycol | 5,00 |
| Acrylates/ C10-30 Alkyl Acrylate Crosspolymer | 0,35 |
| Phenylbenzimidazole Sulfonic Acid | 4,00 |
| Tromethamine (20 %-ige Lösung) | 13,00 |
| Ethylhexyl Salicylate | 5,00 |
| C 12-15 Alkyl Benzoate | 16,0 |
| Ethylhexyl Triazone | 3,00 |
| Butyl Methoxydibenzoylmethane | 5,00 |
| Bis-Ethylhexyloxyphenol Methoxyphenol Triazine | 2,00 |
| Titanium Dioxide, Trimethoxycaprylylsilane | 9,00 |
| Alcohol Denat. | 3,00 |

Die emulgatorfreie Hydrodispersion gemäß des Ausführungsbeispiels wird wie folgt hergestellt:

In einer Wasserphase werden Wasser, EDTA und Pentylene Glycol vorgelegt und darin die Gelbildner Acrylates/C10-30 Alkyl Acrylate Crosspolymer sowie Xanthan Gum dispergiert. Die Mischung wird erwärmt und anschließend Phenylbenzimidazole Sulfonic Acid mit Tromethamine neutralisiert und ebenfalls der Wasserphase zugesetzt.

In einem anderen Ansatzkessel wird die Filterphase zusammen mit Alkyl Benzoate gemischt bzw. gelöst und die/das Pigment(e) eindispergiert.

Die beiden Phasen werden in der Wärme unter Homogenisieren vereinigt und abgekühlt. Bei ca. 40 °C wird ggf. Ethanol zugesetzt, erneut homogenisiert und anschließend auf Raumtemperatur abgekühlt.

Beispiel 2:

| INCI | g/100 g |
|---|---|
| Aqua | 25,05 |
| Disodium EDTA | 0,10 |
| Xanthan Gum | 0,50 |
| Pentylene Glycol | 5,00 |
| Acrylates/ C10-30 Alkyl Acrylate Crosspolymer | 0,35 |
| Phenylbenzimidazole Sulfonic Acid | 4,00 |
| Tromethamine (20 %-ige Lösung) | 13,00 |
| Ethylhexyl Salicylate | 5,00 |
| C 12-15 Alkyl Benzoate | 20,0 |
| Ethylhexyl Triazone | 3,00 |
| Butyl Methoxydibenzoylmethane | 5,00 |
| Bis-Ethylhexyloxyphenol Methoxyphenol Triazine | 2,00 |
| Diethylamino Hydroxybenzoyl Hexyl Benzoate | 5,00 |
| Titanium Dioxide, Trimethoxycaprylylsilane | 9,00 |

(fortgesetzt)

| INCI | g/100 g |
|---|---|
| Alcohol Denat. | 3,00 |

Beispiel 3:

| INCI | g/100 g |
|---|---|
| Aqua | 20,05 |
| Disodium EDTA | 0,10 |
| Xanthan Gum | 0,50 |
| Pentylene Glycol | 5,00 |
| Acrylates/ C10-30 Alkyl Acrylate Crosspolymer | 0,35 |
| Phenylbenzimidazole Sulfonic Acid | 4,00 |
| Tromethamine (20 %-ige Lösung) | 13,00 |
| Ethylhexyl Salicylate | 5,00 |
| C 12-15 Alkyl Benzoate | 20,0 |
| Ethylhexyl Triazone | 3,00 |
| Butyl Methoxydibenzoylmethane | 5,00 |
| Bis-Ethylhexyloxyphenol Methoxyphenol Triazine | 2,00 |
| Diethylamino Hydroxybenzoyl Hexyl Benzoate | 10,00 |
| Titanium Dioxide, Trimethoxycaprylylsilane | 9,00 |
| Alcohol Denat. | 3,00 |

Beispiel 4:

| INCI | g/100 g |
|---|---|
| Aqua | 24,05 |
| Disodium EDTA | 0,10 |
| Xanthan Gum | 0,50 |
| Pentylene Glycol | 5,00 |
| Acrylates/ C10-30 Alkyl Acrylate Crosspolymer | 0,35 |
| Phenylbenzimidazole Sulfonic Acid | 4,00 |
| Tromethamine (20 %-ige Lösung) | 13,00 |
| Ethylhexyl Salicylate | 5,00 |
| C 12-15 Alkyl Benzoate | 20,0 |
| Ethylhexyl Triazone | 3,00 |
| Butyl Methoxydibenzoylmethane | 5,00 |
| Bis-Ethylhexyloxyphenol Methoxyphenol Triazine | 2,00 |
| Diethylamino Hydroxybenzoyl Hexyl Benzoate | 5,00 |
| Titanium Dioxide, Trimethoxycaprylylsilane | 9,00 |
| Zinkoxid | 1,00 |

(fortgesetzt)

| INCI | g/100 g |
|---|---|
| Alcohol Denat. | 3,00 |

Die Hydrodispersionen gemäß den Beispielen 2-4 werden entsprechend der Vorschrift zu Beispiel 1 hergestellt.

**Patentansprüche**

1. Lichtschutzzubereitung in Form einer Hydrodispersion,
**dadurch gekennzeichnet, dass** sie mindestens einen öllöslichen, mindestens einen wasserlöslichen und mindestens einen pigmentären UV-Filter enthält und einen Lichtschutzfaktor von ≥ 50 aufweist.

2. Lichtschutzzubereitung gemäß Anspruch 1,
**dadurch gekennzeichnet, dass** der Emulgatorgehalt ≤ 2 Gew.-%, bevorzugt ≤ als 0,2 Gew.-% und besonders bevorzugt ≤ 0,01 Gew.-% ist.

3. Lichtschutzzubereitung gemäß einem der Ansprüche 1 oder 2,
**dadurch gekennzeichnet, dass** sie emulgatorfrei ist.

4. Lichtschutzzubereitung gemäß einem der Ansprüche 1-3,
**dadurch gekennzeichnet, dass** der Fettgehalt ≤ 1 Gcw.-%, bevorzugt ≤ 0,1 Gew.-% und besonders bevorzugt ≤ 0,01 Gew,-% ist.

5. Lichtschutzzubereitung gemäß einem der Ansprüche 1-4,
**dadurch gekennzeichnet, dass** sie fettfrei ist.

6. Lichtschutzzubereitung gemäß einem der Ansprüche 1-5,
**dadurch gekennzeichnet, dass** der pigmentäre UV-Filter ein anorganischer pigmentäter UV-Filter ist.

7. Lichtschutzzubereitung gemäß einem der Ansprüche 1-6,
**dadurch gekennzeichnet, dass** sie für die Verwendung zum Schutz der Haut vor Sonnenstrahlen, insbesondere UV-Strahlen, bei Sonnenallergie und/oder Mallorca-Akne geeignet ist.

8. Lichtschutzzubereitung gemäß einem der Ansprüche 1-7,
**dadurch gekennzeichnet, dass** der oder die wasserlöslichen UV-Filter in einer Menge von 0,1 bis 10 Gew.-%, insbesondere von 0,5 bis 6 Gew.-% vorliegen.

9. Lichtschutzzubereitung gemäß einem der Ansprüche 1-8,
**dadurch gekennzeichnet, dass** der oder die öllöslichen UV-Filter jeweils in einer Menge von 0,1 bis 10 Gew.-%, insbesondere von 1 bis 7 Gew.-% vorliegen.

10. Lichtschutzzubereitung gemäß einem der Ansprüche 1-9,
**dadurch gekennzeichnet, dass** der oder die pigmentären UV-Filter in einer Menge von 0,1 bis 25 Gew.-%, insbesondere von 0,5 bis 12 Gew.-% vorliegen.

11. Lichtschutzzubereitung gemäß einem der Ansprüche 1-10,
**dadurch gekennzeichnet, dass** das Verhältnis von löslichen zu pigmentären UV-Filtern im Bereich von 0,2 bis 10, insbesondere von 0,5 bis 5 liegt.

12. Lichtschutzzubereitung gemäß einem der Ansprüche 1-11,
**dadurch gekennzeichnet, dass** das Verhältnis der wasserlöslichen zu den öllöslichen UV-Filtern im Bereich von 0,02 bis 2, insbesondere von 0,1 bis 1 liegt,

13. Lichtschutzzubereitung gemäß einem der Ansprüche 1-12,
**dadurch gekennzeichnet, dass** sie frei von Konservierungsstoffen ist.

**14.** Liehtschutzzubereitung gemäß einem der Ansprüche 1-13,
**dadurch gekennzeichnet, dass** sie frei von Parfümölen ist.

**15.** Lichtschutzzubereitung gemäß einem der Ansprüche 1-14,
**dadurch gekennzeichnet, dass** sie mindestens einen UV-A-Filter enthält.

**16.** Lichtschutzzubereitung gemäß Anspruch 15,
**dadurch gekennzeichnet, dass** sie Butylmethoxydibenzoylmethane und/oder Diethylamino Hydroxybenzoyl Hexyl Benzoate und/oder Bis-Ethylhexyloxyphenol Mehoxyphenyl Triazine enthält.

**17.** Lichtschutzzubereitung gemäß einem der Ansprüche 1-16,
**dadurch gekennzeichnet, dass** sie einen UV-A-Schutzfaktor von $\geq 20$ aufweist.

**18.** Verwendung einer Lichtschutzzubereitung gemäß einem der Ansprüche 1-17 zum Schutz von gegenüber Sonnenallergie und/oder Mallorca-Akne empfindlicher und/oder von Sonnenallergie und/oder Mallorca-Akne betroffener Haut vor UV-Strahlutug.

## EUROPÄISCHER RECHERCHENBERICHT

**Europäisches Patentamt**

**Nummer der Anmeldung**

EP 08 00 3246

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 94/17780 A (BEIERSDORF AG [DE]; GERS BARLAG HEINRICH [DE]; HACHMANN STEFAN [DE]; N) 18. August 1994 (1994-08-18) * Seite 5, Zeilen 26-32; Ansprüche; Beispiele 6-8 * ----- | 1-18 | INV. A61K8/04 A61K8/29 A61K8/35 A61K8/41 A61K8/46 A61K8/49 A61Q17/04 |
| X | DE 101 35 258 A1 (BEIERSDORF AG [DE]) 6. Februar 2003 (2003-02-06) * Seite 13 - Seite 14; Beispiele 2-5 * ----- | 1-18 | |
| X | EP 1 277 460 A (BEIERSDORF AG [DE]) 22. Januar 2003 (2003-01-22) * Seite 12 - Seite 13; Beispiele 2-4 * ----- | 1-18 | |
| X | EP 1 752 135 A (BEIERSDORF AG [DE]) 14. Februar 2007 (2007-02-14) * Seite 17 - Seite 18; Beispiele 1,3,6 * ----- | 1-18 | |
| X | DE 101 40 547 A1 (BEIERSDORF AG [DE]) 27. Februar 2003 (2003-02-27) * Seite 11 - Seite 12; Beispiele 1,4 * ----- | 1-18 | RECHERCHIERTE SACHGEBIETE (IPC) |
| X | EP 1 285 648 A (BEIERSDORF AG [DE]) 26. Februar 2003 (2003-02-26) * Seite 11 - Seite 12; Beispiele 1,4 * ----- | 1-18 | A61K A61Q |
| X | DE 101 41 473 A1 (BEIERSDORF AG [DE]) 20. März 2003 (2003-03-20) * Seite 13 - Seite 14; Beispiele 1-5 * ----- | 1-18 | |
| X | DE 101 55 716 A1 (BEIERSDORF AG [DE]) 22. Mai 2003 (2003-05-22) * Seite 17 - Seite 18; Beispiele 1-5 * ----- | 1-18 | |
| X | DE 101 38 499 A1 (BEIERSDORF AG [DE]) 13. Februar 2003 (2003-02-13) * Seite 29 - Seite 30; Beispiele 1,3-5 * ----- | 1-18 | |

-/--

Der vorliegende Recherchenbericht wurde für alle Patentansprüche erstellt

| Recherchenort | Abschlußdatum der Recherche | Prüfer |
|---|---|---|
| München | 29. Juli 2008 | Mitchell, Gemma |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

**Europäisches Patentamt**

**EUROPÄISCHER RECHERCHENBERICHT**

Nummer der Anmeldung

EP 08 00 3246

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 281 390 A (BEIERSDORF AG [DE]) 5. Februar 2003 (2003-02-05) * Seite 34 - Seite 35; Beispiele 1,4,5 * ----- | 1-18 | |
| X | DE 195 30 574 A1 (BASF AG [DE]) 20. Februar 1997 (1997-02-20) * Ansprüche 10,11 * ----- | 18 | |
| X | EP 1 579 847 A (BEIERSDORF AG [DE]) 28. September 2005 (2005-09-28) * Absatz [0023]; Ansprüche 1-10,26 * ----- | 18 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 29. Juli 2008 | Mitchell, Gemma |

EPO FORM 1503 03.82 (P04C03)

# EP 2 092 928 A1

**ANHANG ZUM EUROPÄISCHEN RECHERCHENBERICHT
ÜBER DIE EUROPÄISCHE PATENTANMELDUNG NR.**

EP 08 00 3246

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-07-2008

| Im Recherchenbericht angeführtes Patentdokument | | Datum der Veröffentlichung | Mitglied(er) der Patentfamilie | | Datum der Veröffentlichung |
|---|---|---|---|---|---|
| WO 9417780 | A | 18-08-1994 | AT<br>EP<br>ES<br>JP<br>US | 167392 T<br>0683662 A1<br>2118381 T3<br>8506574 T<br>5725844 A | 15-07-1998<br>29-11-1995<br>16-09-1998<br>16-07-1996<br>10-03-1998 |
| DE 10135258 | A1 | 06-02-2003 | KEINE | | |
| EP 1277460 | A | 22-01-2003 | DE | 10135024 A1 | 24-04-2003 |
| EP 1752135 | A | 14-02-2007 | DE 102005037546 A1 | | 22-02-2007 |
| DE 10140547 | A1 | 27-02-2003 | EP | 1306080 A1 | 02-05-2003 |
| EP 1285648 | A | 26-02-2003 | AT<br>DE | 348592 T<br>10140546 A1 | 15-01-2007<br>06-03-2003 |
| DE 10141473 | A1 | 20-03-2003 | WO<br>EP<br>US | 2004056333 A1<br>1453478 A1<br>2004258638 A1 | 08-07-2004<br>08-09-2004<br>23-12-2004 |
| DE 10155716 | A1 | 22-05-2003 | KEINE | | |
| DE 10138499 | A1 | 13-02-2003 | WO<br>EP | 03013455 A2<br>1416912 A2 | 20-02-2003<br>12-05-2004 |
| EP 1281390 | A | 05-02-2003 | DE | 10138496 A1 | 20-02-2003 |
| DE 19530574 | A1 | 20-02-1997 | AU<br>AU<br>CN<br>WO<br>EP<br>ES<br>JP<br>US | 711515 B2<br>6820296 A<br>1199384 A<br>9707058 A1<br>0844980 A1<br>2153972 T3<br>11513054 T<br>6074472 A | 14-10-1999<br>12-03-1997<br>18-11-1998<br>27-02-1997<br>03-06-1998<br>16-03-2001<br>09-11-1999<br>13-06-2000 |
| EP 1579847 | A | 28-09-2005 | DE 102004020627 A1<br>US 2006008426 A1 | | 13-10-2005<br>12-01-2006 |

EPO FORM P0461

Für nähere Einzelheiten zu diesem Anhang : siehe Amtsblatt des Europäischen Patentamts, Nr.12/82